# FASCICULE DE BREVET EUROPEEN

(11) **EP 4 284 256 B1**
(45) Date de publication et mention de la délivrance du brevet: **29.10.2025**
(21) Numéro de dépôt: 22705859.1
(22) Date de dépôt: 01.02.2022
(51) Int. Cl.: A61B 17/02

(54) **ECARTEUR CHIRURGICAL**
CHIRURGISCHER RETRAKTOR
SURGICAL RETRACTOR

(30) Priorité: 01.02.2021 US 202163144156 P; 02.04.2021 FR 2103425
(43) Date de publication de la demande: 06.12.2023
(73) Titulaire: Clariance, 62217 Beaurains (FR)
(72) Inventeur: KRIER, Brice, 62128 St. Leger (FR)
(74) Mandataire: Leone, Eloïse
(86) Numéro de dépôt international: PCT/FR2022/050177
(87) Numéro de publication internationale: WO 2022/162327

(56) Documents cités:
- WO-A1-01/03586
- US-A1- 2009 299 148
- US-A1- 2010 274 094
- US-A1- 2011 301 421
- US-A1- 2012 271 336
- US-A1- 2015 018 625
- US-A1- 2017 196 548

## Description

### DOMAINE D'APPLICATION DE L'INVENTION

L'invention a trait au domaine des écarteurs chirurgicaux et notamment aux adaptations permettant d'améliorer les fonctionnalités desdits écarteurs.

### DESCRIPTION DE L'ART ANTERIEUR

Dans le domaine de la chirurgie notamment mini-invasive, il est connu d'utiliser des écarteurs chirurgicaux qui assurent l'écartement des tissus du patient pour proposer un accès sans lésion au praticien.

Parmi les écarteurs chirurgicaux, il existe des écarteurs adoptant une géométrie concentrique comprenant des lames mobiles (aussi appelées valves) se déplaçant concentriquement dont l'écartement crée entre elles le chemin d'accès

Parmi ces écarteurs, le document WO0103586 décrit un dispositif écarteur à aiguilles ou lames extensible, comprenant une base conçue aux fins de positionnement sur une ouverture pratiquée dans un partie corporelle, et un élément écarteur à forme d'aiguille extensible comportant un axe longitudinal parcouru par un orifice, dont la configuration permet l'insertion via l'ouverture de façon à pénétrer la partie corporelle et engager ainsi un tissu corporel. L'élément écarteur possède une première extrémité supportée en association avec la base et une seconde extrémité, libre, séparée axialement de la base. Un appareil d'expansion, monté sur la base, permet de déployer radialement l'élément écarteur, de manière réglable autour de l'axe longitudinal, afin d'écarter un tissu engagé par l'élément écarteur, permettant ainsi de dégager une zone de travail à l'intérieur de la partie corporelle, en alignement généralement axial avec l'ouverture.

La base sert alors de support et de guidage à une roue d'entraînement commandée par l'utilisateur. Par un moyen de transmission, la rotation de la roue provoque la translation radiale d'une pluralité de bras. Chaque bras est associé à une aiguille de sorte que la rotation dans un sens ou dans l'autre de la roue d'entraînement provoque l'écartement ou le rapprochement synchronisé des lames.

Un tel écarteur est encombrant. De plus, la commande de la rotation de la roue est réalisée au moyen d'au moins un bouton rotatif disposé sur la base. En conséquence, l'accessibilité visuelle est réduite lorsque le chirurgien actionne le bouton.

En outre, la rotation du bouton doit assurer la rotation de la roue dont le mouvement de rotation doit être transformé par un système de molette en mouvement de translation pour les bras. Un tel dispositif requiert donc des moyens complexes de transmission multipliant le nombre d'éléments constitutifs.

Il existe également dans l'art antérieur, des écarteurs à géométrie concentrique qui autorisent le déplacement désynchronisé des bras. La mise en œuvre d'une telle fonction requiert alors des moyens d'embrayage et des moyens séparés de commande qui complexifient et rendent plus encombrant l'écarteur. US2017196548 A1 décrit un écarteur selon le préambule de la revendication 1.

### BREVE DESCRIPTION DE L'INVENTION

Ce que constatant, la demanderesse a mené des recherches visant à améliorer les écarteurs chirurgicaux.

Les recherches ont abouti à la conception et à la réalisation d'un écarteur chirurgical nouveau plus ergonomique, moins encombrant et présentant des fonctionnalités nouvelles.

Cet écarteur chirurgical comprend :
- un cadre présentant une ouverture centrale,
- des bras mobiles en translation,
- une lame associée à chaque bras,

Les bras présentant deux extrémités :
Une première extrémité coopérant avec le cadre à des fins de guidage et d'entraînement et une deuxième extrémité évoluant dans l'ouverture centrale ;

Les lames présentant deux extrémités :
Une première extrémité en liaison avec la deuxième extrémité d'un bras et une deuxième extrémité destinée à coopérer avec les tissus du patient.

Selon l'invention, l'écarteur est remarquable en ce qu'il comprend deux portions de roues indépendantes guidées en rotation par le cadre et venant coopérer avec des bras différents pour proposer des mises en mouvement indépendantes.

Cette caractéristique est particulièrement avantageuse en ce qu'au moins un bras (et donc une lame) est commandable de façon séparée. La mise en mouvement séparée est réalisée par le fait qu'il n'y a plus seulement une roue de mise en mouvement mais deux portions de roue séparées. **Il** n'est plus nécessaire d'utiliser un moyen d'embrayage complexe pour autoriser ces mouvements non synchronisés.

Un mode d'utilisation privilégié de cet écarteur est pour la chirurgie de la colonne vertébrale. Il permet notamment l'accès à la colonne en écartant les tissus et les viscères par exemple par voie latérale ou antérieure.

Lors de son utilisation, l'écarteur peut être lié à un bras chirurgical articulé tel celui dit de Martin au moyen de son cadre ou au moyen d'un de ses bras.

Pour ce faire, selon une autre caractéristique particulièrement avantageuse de l'invention, le cadre est équipé d'un moyen de liaison mécanique préformé d'un orifice de fixation permettant une fixation audit bras.

De même, selon une autre caractéristique particulièrement avantageuse de l'invention, la première extrémité d'au moins un bras est équipée d'un orifice restant accessible en toute position (même rétractée) permettant la liaison avec un bras chirurgical.

La lame séparée peut être fixée et/ou prendre appui sur le corps du patient et c'est l'écarteur qui se déplace par rapport à la lame. Elle peut également ne pas être fixée ou ne pas prendre appui. Elle se déplace alors par rapport au cadre de l'écarteur.

Selon l'invention, les deux portions de roues ne sont pas coaxiales. Cette caractéristique permet de proposer un cadre et donc un écarteur moins encombrant. En effet, des portions de roues concentriques se seraient chevauchées, ce qui aurait eu pour effet d'augmenter l'épaisseur de l'écarteur, ce qui n'est pas le cas avec des portions de roues non concentriques.

Les portions de roues peuvent transmettre le mouvement par tout moyen par exemple par adhérence. Néanmoins, selon un mode de réalisation préféré mais non limitatif, les portions de roues sont des portions de roues dentées.

Selon un mode de réalisation préféré mais non limitatif, les bras et les lames associées sont au nombre de trois disposés à 120 degrés autour de l'axe du cylindre formé par les lames réunies en position rétractée.

Selon une autre caractéristique particulièrement avantageuse de l'invention, chaque portion de roue est équipée d'un doigt de commande mobile se projetant hors du cadre, doigt déplaçable par l'utilisateur. Les doigts de déplacements permettent d'avoir la même fonction qu'une molette que l'on peut retrouver dans d'autres dispositifs, mais en limitant le nombre de pièces, donc d'augmenter la robustesse du système.

L'ergonomie est améliorée car le déplacement induit du doigt permet plus de rapidité de déplacement des lames pour le chirurgien. De plus, le mouvement est intuitif car une translation du doigt induit une translation de lame et le retour de l'effort est plus direct.

Selon une autre caractéristique particulièrement avantageuse, un doigt fixe est fixé au cadre en regard de la course de chaque doigt mobile. Cette disposition donne un appui aux doigts de l'utilisateur des doigts mobiles et permet de reproduire pour l'utilisateur les mouvements de la main réalisés lors de l'utilisation d'une pince ce qui optimise encore l'ergonomie.

Le fait d'avoir des doigts mobiles permet de décaler l'action des mains de l'utilisateur et de les positionner à l'extérieur du cadre et donc dans une position éloignée de la plaie. Cette position permet en outre de garder une meilleure vision de la zone opérée.

Selon une autre caractéristique particulièrement avantageuse de l'invention, l'écarteur comprend en outre un moyen de maintien en position des lames.

Pour mettre en œuvre cette fonction, chaque doigt mobile est associé à un curseur qui a un comportement de cliquet en autorisant la rotation de la portion de roue dentée dans un sens (le sens correspondant à l'écartement des lames) et en bloquant la rotation dans un autre sens. Un désengagement du cliquet commandé par l'utilisateur autorise la rotation en sens inverse et donc la rétractation des lames.

Selon un mode de réalisation préféré mais non limitatif, le curseur lié au doigt mobile est monté sur une lame ressort et comprend au moins une pointe qui vient coopérer avec des dentures orientées associées au cadre fixe.

Selon une autre caractéristique particulièrement avantageuse de l'invention, la première extrémité des lames est préformée de façon à accueillir un module fonctionnel.

Pour ce faire selon une autre caractéristique, la première extrémité des lames est préformée d'au moins un orifice débouchant sur la surface interne de la lame.

Cet orifice forme un canal donnant un accès à la zone écartée.

Par exemple, il constitue un puits de lumière lorsque la lame accueille un module fonctionnel de type moyen lumineux.

Il permet également un accès à des moyens vidéos lorsque la lame accueille une caméra endoscopique.

Selon une autre caractéristique particulièrement avantageuse de l'invention, au moins les parties supérieures des bords longitudinaux des lames présentent des profils complémentaires d'emboîtement optimisant la liaison entre elles une fois en contact.

Selon une autre caractéristique particulièrement avantageuse de l'invention, les lames sont disponibles dans différentes longueurs. L'écarteur est associé à une pluralité de jeux de lames pour former un kit. Ainsi, le chirurgien a la possibilité de sélectionner les lames les plus adéquates à la morphologie du patient opéré.

Selon une autre caractéristique particulièrement avantageuse de l'invention, les lames sont fabriquées dans un matériau radio transparent.

Selon une autre caractéristique particulièrement avantageuse de l'invention, les lames sont en titane et leur épaisseur est affinée afin de les rendre radiotransparentes.

Selon une autre caractéristique particulièrement avantageuse de l'invention, la liaison entre la première extrémité des lames et la deuxième extrémité des bras autorise une inclinaison. Les lames peuvent être inclinées (par exemple jusqu'à quinze degrés).

Pour ce faire, la lame est associée à une platine montée pivotante par rapport au bras. La platine assure dont une fonction de pièce de liaison intermédiaire entre le bras et la lame.

Selon un mode de réalisation non limitatif, en dehors des lames, les autres éléments constitutifs sont fabriqués dans des matériaux choisis dans la liste non exhaustive suivante :
- alliage de titane,
- acier inoxydable,
- tout type de polymères ;
- aluminium ou alliage d'aluminium.

Les concepts fondamentaux de l'invention venant d'être exposés ci-dessus dans leur forme la plus élémentaire, d'autres détails et caractéristiques ressortiront plus clairement à la lecture de la description qui suit et en regard des dessins annexés, donnant à titre d'exemple non limitatif, un mode de réalisation d'un écarteur conforme à l'invention.

### BRÈVE DESCRIPTION DES DESSINS

[Fig. 1] est un dessin schématique d'une vue de dessus d'un mode de réalisation d'un écarteur conforme à l'invention en position rétractée ;

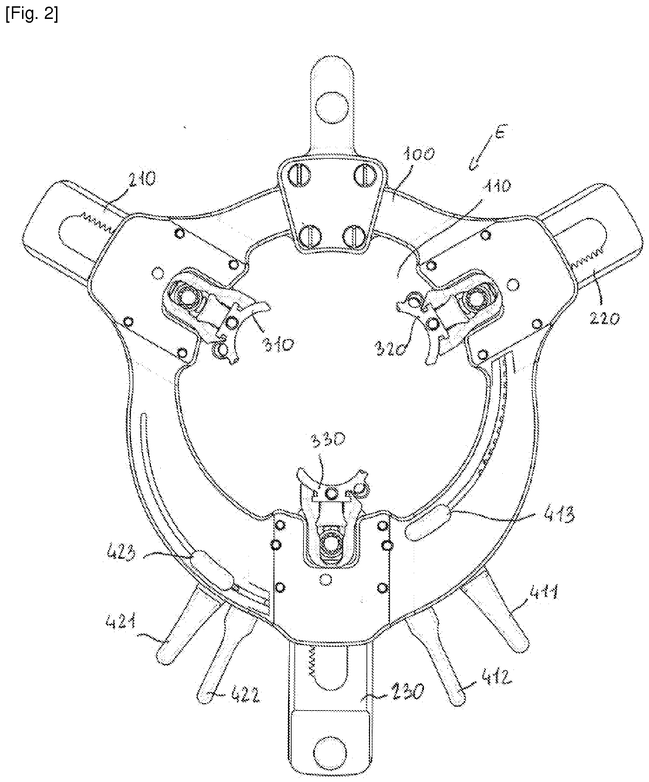

[Fig. 2] est un dessin schématique d'une vue de dessus de l'écarteur de la figure 1 en position écartée ;

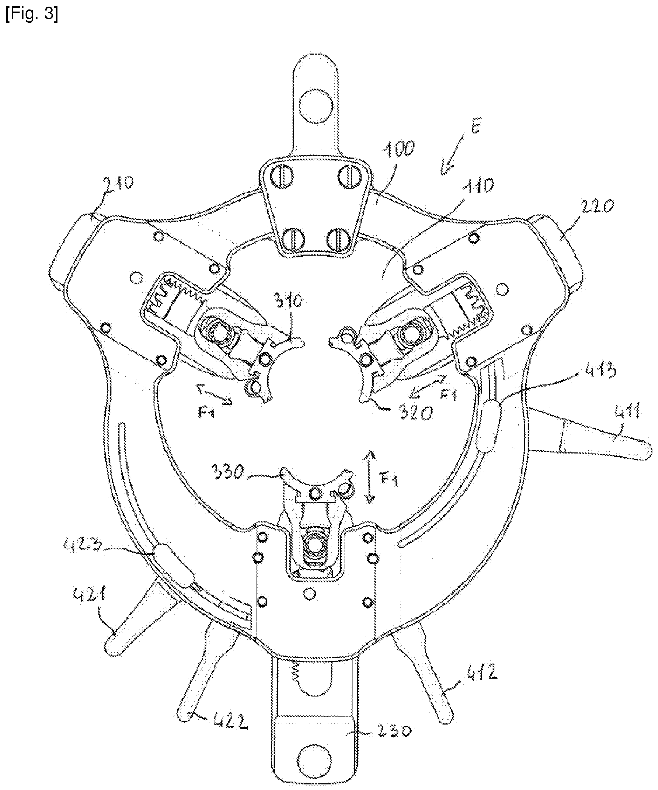

[Fig. 3] est un dessin schématique d'une vue de dessus de l'écarteur de la figure 1 avec des positions de lames différentes ;
[Fig. 4] est un dessin schématique d'une vue de dessus partiellement éclatée de l'écarteur de la figure 1 ;
[Fig. 5] est un dessin schématique d'une vue de dessus du seul cadre de l'écarteur de la figure 1 ;

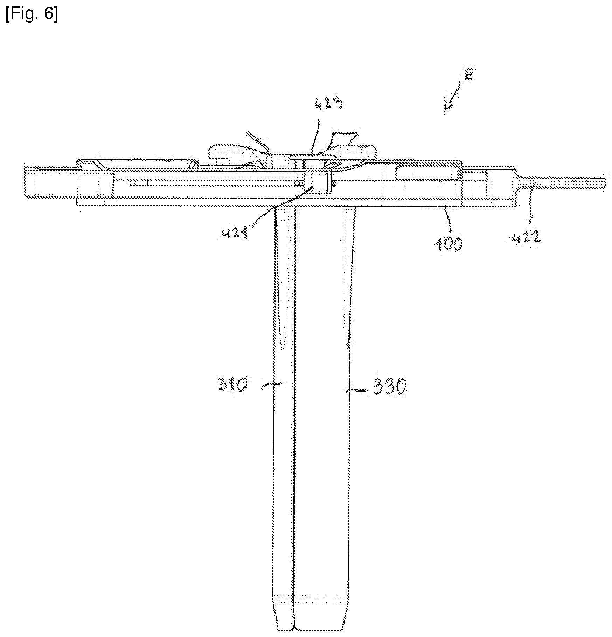

[Fig. 6] est un dessin schématique d'une vue de côté de de l'écarteur de la figure 1 en position rétractée ;
[Fig. 7] est un dessin schématique d'une vue en perspective du détail de la liaison entre la première extrémité d'une lame et la deuxième extrémité d'un bras ;
[Fig. 8] est un dessin schématique d'une vue en coupe de l'écarteur de la figure 1 au niveau d'un des curseurs équipant l'écarteur.

### DESCRIPTION D'UN MODE PRÉFÉRÉ DE RÉALISATION

Comme illustré par les figures, l'écarteur chirurgical référencé E dans son ensemble comprend un cadre 100 adoptant une géométrie sensiblement annulaire et présentant une ouverture centrale 110.

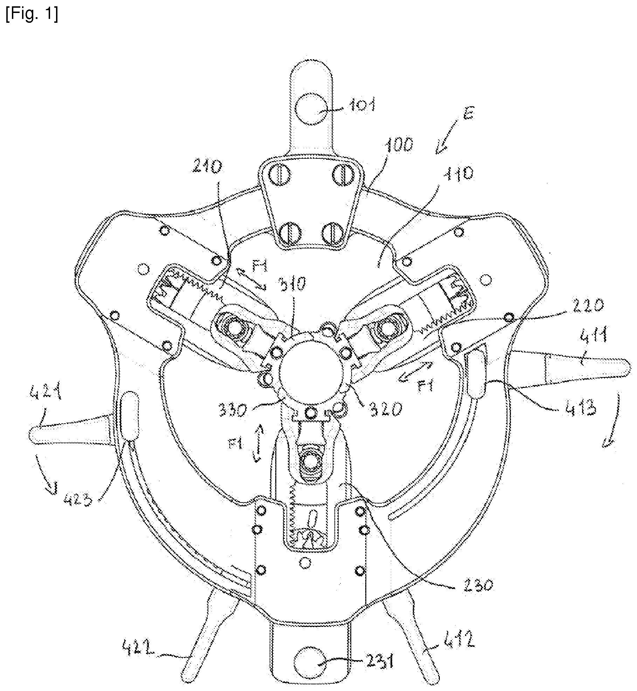

Ce cadre 100 supporte trois bras 210, 220, 230 mobiles en translation (selon la double flèche F1) et disposés à 120 degrés sur ledit cadre 100. Une lame 310, 320, 330 est associée à chaque bras. Les trois lames sont identiques et une fois en contact (cf. figure 1) se complètent pour former un cylindre.

Plus précisément, selon le mode de réalisation illustré, au moins la partie supérieure des bords longitudinaux des lames présente un profil complémentaire d'emboîtement optimisant la liaison entre elles une fois en contact.

Les bras 210, 220, 230 présentent deux extrémités :
Une première extrémité externe coopérant avec le cadre 100 à des fins de guidage et d'entraînement et une deuxième extrémité interne évoluant dans l'ouverture centrale 110.

Les lames 310, 320, 330 présentent deux extrémités :
Une première extrémité haute en liaison avec la deuxième extrémité interne d'un bras et une deuxième extrémité basse destinée à coopérer avec les tissus du patient.

Lors de la mise en place sur le patient, les lames 310, 320, 330 sont passées autour de dilatateurs (non illustrés) préalablement insérés dans les tissus du patient. La longueur des lames est choisie selon des graduations présentes sur le dernier dilatateur.

L'écarteur E peut être fixé sur une table d'opération au moyen d'un bras chirurgical dit de Martin non illustré. Le cadre 100 est équipé d'un moyen de liaison mécanique préformé d'un orifice de fixation 101 permettant une fixation audit bras.

Comme illustrée, la première extrémité d'au moins un bras, ici le bras 230, est équipée d'un orifice 231 restant accessible en toute position (même rétractée) et permettant également la liaison avec un bras chirurgical.

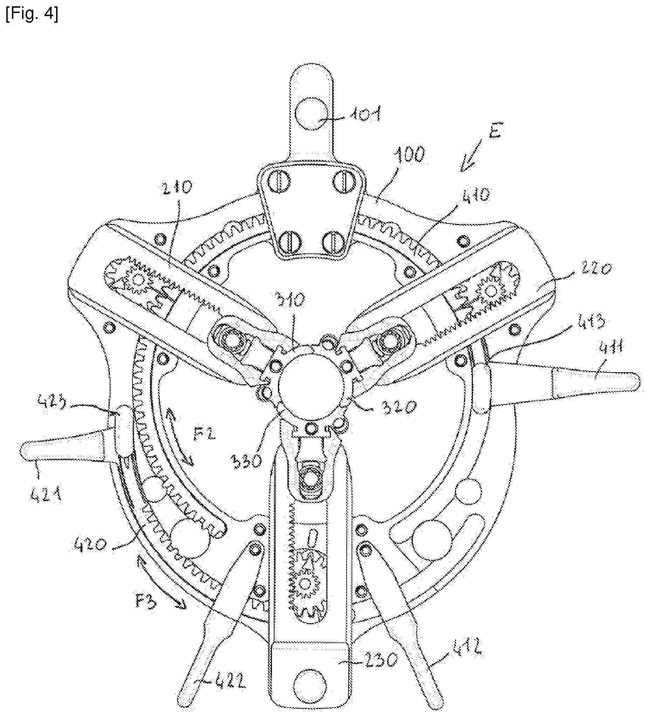

Comme illustré plus en détail sur la figure 4, l'écarteur E comprend deux portions de roues dentées 410 et 420 indépendantes et non coaxiales guidées en rotation par le cadre 100 qui est préformé à cet effet et venant coopérer avec des bras différents pour proposer des mises en mouvement indépendantes.

Ainsi, la portion de roue dentée 410 commande par son mouvement de rotation (double flèche F2) la translation (double flèche F1) des bras 210 et 220.

La portion de roue dentée 420 commande par son mouvement de rotation (double flèche F3) la translation (double flèche F1) du bras 230.

Comme illustrées, les portions de roues dentées 410 et 420 ne sont pas concentriques mais les axes des translations horizontales selon la double flèche F1 sont concourants.

La commande séparée d'un des bras (ici le bras 230) permet un fonctionnement plus souple de l'écarteur E. Il permet notamment d'envisager une fixation de l'écarteur E sur le corps du patient par l'intermédiaire de l'extrémité de la lame 330.

Selon le mode de réalisation illustré, chaque bras est monté glissant par rapport au cadre 100 et est aménagé d'une lumière longitudinale oblongue préformée sur un côté d'une crémaillère. Cette crémaillère coopère avec un pignon de petit diamètre coaxial et solidaire en rotation d'un pignon de plus grand diamètre monté pivotant sur le cadre 100 et coopérant avec une portion de roue dentée.

Selon un mode de réalisation préféré mais non limitatif, pour une course de bras comprise entre 150 et 300 millimètres :
- les portions de roue dentée comprennent entre 30 et 50 dents, et
- les pignons doubles comprennent sur leur petit et grand diamètre entre 10 et 20 dents.

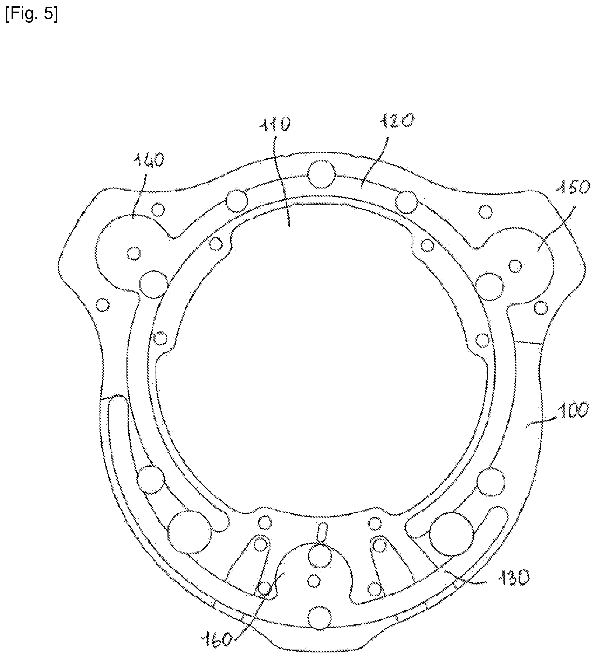

Le cadre 100 est formé d'une partie supérieure et d'une partie inférieure. La figure 5 illustre la partie inférieure du cadre 100 préformée de rainures 120 et 130 guidant la rotation des portions de roue dentée respectivement 410 et 420. Le cadre est également préformé de logements 140,150 et 160 des pignons de grand diamètre. Le volume creux de la rainure 120 guidant la portion de roue dentée 410 rejoint le volume creux des logements 140 et 150 destiné à l'accueil des pignons de grand diamètre commandant la translation des bras 210 et 220.

Le volume creux de la rainure 130 guidant la portion de roue dentée 420 rejoint le volume creux du logement 160 destiné à l'accueil du pignon de grand diamètre commandant la translation du bras indépendant 230.

Comme illustrées, les deux portions de roues dentées 410 et 420 évoluent dans le même plan ce qui est rendu possible par leur non coaxialité. Le cadre et donc l'écarteur E sont ainsi compacts et légers.

Comme illustrée, chaque portion de roue 410 et 420 est équipée d'un doigt de commande mobile 411 et 421 se projetant selon une direction rayonnante hors du cadre 100, doigt déplaçable par l'utilisateur. Un doigt fixe 412, 422 est fixé au cadre 100 en regard de la course de chaque doigt mobile 411 et 421.

Ce doigt fixe permet à l'utilisateur de prendre appui pour actionner le doigt mobile. Ainsi, l'utilisateur prend appui sur le doigt fixe 412 pour actionner le doigt mobile 411 et commander la translation des bras 210 et 220. De même, il prend appui sur le doigt fixe 422 pour actionner le doigt mobile 421 et commander la translation du bras 230.

Un curseur 413 et 423 est associé à chaque doigt mobile 411 et 421 et se déplace sur la surface supérieure du cadre 100.

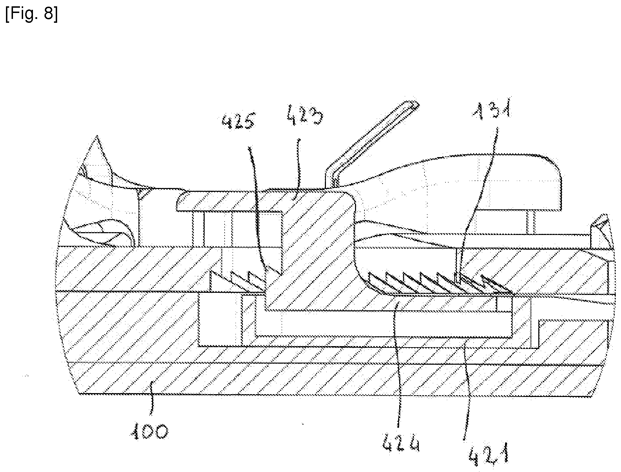

Comme illustré par la figure 8 pour le curseur 423, il est monté sur une lame élastique 424 et est préformé pour servir de cliquet, c'est-à-dire qu'il autorise la rotation de la portion de roue dentée auquel il est associé dans un seul sens.

L'utilisateur doit appuyer sur ce curseur 423 pour autoriser le déplacement dans l'autre sens. Ces curseurs servent donc de moyen de maintien en position des lames.

Plus précisément, une extrémité du curseur mobile 423 est constituée d'une pointe chanfreinée 425 qui évolue dans le cadre 100. Cette pointe 425 est en contact et se bloque dans des dentures 131 qui sont associées au cadre 100 fixe.

L'orientation des dentures 131 autorise le déplacement dans un sens (correspondant à celui de l'écartement des lames) et empêche le déplacement dans l'autre (correspondant à la rétractation).

L'appui sur le curseur 423 provoque un déplacement de la pointe 425 autorisé par la lame élastique 424, ce qui assure le désengagement entre la pointe 425 et les dentures 131.

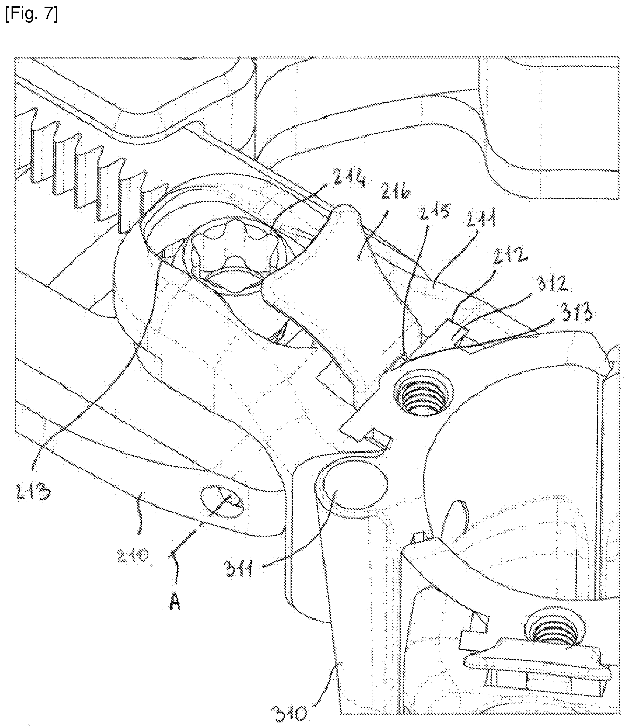

Comme illustrée par la figure 7, l'extrémité haute des lames (ici la lame 310) est articulée audit bras 210 de façon à autoriser un pivotement et le maintien d'une position inclinée.

Cette fonctionnalité est ici mise en œuvre au moyen d'une platine 211 montée pivotante par rapport à la deuxième extrémité du bras 210 et comprenant deux extrémités :
- - une première extrémité préformée d'une rainure 212 d'accueil d'une nervure 312 préformée en surface externe de la lame 310,
- - une deuxième extrémité préformée d'un trou oblong 213 dans lequel prend appui la tête d'une vis 214 vissée à la deuxième extrémité du bras.

Le vissage de la vis 214 commande la position angulaire de la platine et donc de la lame par rapport au bras.

L'axe de pivotement référencé A est perpendiculaire à l'axe de translation du bras. En outre, cet axe de pivotement est disposé en avant de la vis d'action 214 ce qui autorise un déplacement angulaire fin de la platine 211 et donc de l'extrémité haute de la lame.

Selon le mode de réalisation préféré mais non limitatif illustré, la nervure 312 comprend en outre un ergot 313. Cet ergot 313 coopère avec une encoche 215 pratiquée dans une lamelle élastique 216 fixée à la platine 211 et assurant la retenue de la lame 310 par rapport à la platine 211. L'élasticité de la lamelle 216 maintient la position de retenue, autorise sans action, l'accueil de la lame 310 dans la rainure 212 et requérant une action de l'utilisateur sur la lamelle 216 pour le démontage de la lame 310.

Cette extrémité haute des lames est en outre conçue de façon à accueillir des modules fonctionnels en étant préformée dans son épaisseur d'au moins un (ici un) orifice 311 pour former un canal ayant un axe différent de celui de la lame de façon à déboucher sur la face interne de la lame. Ce canal est ménagé sur un côté de la lame.

Le module fonctionnel peut être différent ou non pour chaque lame et est sélectionné dans la liste suivante :
- moyen lumineux,
- caméra endoscopique,
- etc...

Ce qui suit est une méthode d'utilisation de l'écarteur E tel que ci-dessus décrit et représenté. À cet égard, il doit être compris que la description suivante est une méthode parmi une pluralité de méthodes différentes dans lesquelles un tel rétracteur peut être utilisé. Pour utiliser l'écarteur E, une incision est faite dans le corps d'un patient. Un premier dilatateur est ensuite inséré dans l'incision pour créer un canal au travers des muscles. D'autres dilatateurs de diamètres successivement plus grands peuvent être insérés par-dessus le premier dilatateur. Les lames 310, 320 et 330 adéquates sont sélectionnées en fonction de la profondeur de pénétration du dernier dilatateur introduit. La lame 310 est assemblée à l'écarteur E en la glissant et en la clipsant dans sa rainure d'accueil 212. Similairement, les lames 320 et 330 sont assemblées à l'écarteur E en les glissant et en les clipsant dans leur rainure d'accueil respective. L'écarteur E avec les lames en position fermée et sans angulation ou inclinaison, est inséré dans le site opératoire par-dessus le dernier dilatateur introduit qui est alors retiré pour créer au travers des muscles un canal libre d'accès à la colonne vertébrale. A ce stade, le ou les modules fonctionnels peuvent être introduit dans les orifices des lames 310, 320 et 330 prévus à cet effet. En prenant appui sur le doigt fixe 422, le doigt mobile 421 est actionné pour commander la translation du bras 230 et de la lame 330. Pour rétracter les deux bras 210 et 220 et les deux lames 310 et 320, le doigt mobile 411 est actionné en prenant appui sur le doigt fixe 412. Pour augmenter l'inclinaison de la lame 310, la vis 214 est vissée dans le sens horaire. Pour relâcher l'inclinaison de la lame 310, la vis 214 est dévissée dans le sens anti-horaire. De façon similaire, l'inclinaison des lames 320 et 330 peut être ajustée chacune indépendamment. De cette façon, les extrémités basses des lames peuvent être écartées sans modifier de façon significative la position des extrémités hautes des lames. Et ainsi une ouverture d'un diamètre plus important, que celui créé par les extrémités hautes, est créée à proximité de l'espace intervertébral à opérer.

On comprend que l'écarteur, qui vient d'être ci-dessus décrit et représenté, l'a été en vue d'une divulgation plutôt que d'une limitation. Bien entendu, divers aménagements, modifications et améliorations pourront être apportés à l'exemple ci-dessus, sans pour autant sortir du cadre de l'invention tel que définit par l'étendue des revendications.

## Revendications

1. Ecarteur chirurgical (E) comprenant :
- un cadre (100) présentant une ouverture centrale (110),
- des bras (210, 220, 230) mobiles en translation,
- une lame (310, 320, 330) associée à chaque bras,
Les bras (210, 220, 230) présentant deux extrémités :
Une première extrémité coopérant avec le cadre (100) à des fins de guidage et d'entraînement et une deuxième extrémité évoluant dans l'ouverture centrale (110),
Les lames (310, 320, 330) présentant deux extrémités :
Une première extrémité en liaison avec la deuxième extrémité d'un bras (210, 220, 230) et une deuxième extrémité destinée à coopérer avec les tissus du patient,
**CARACTERISE PAR LE FAIT QU'**il comprend deux portions de roues indépendantes (410, 420) non coaxiales, guidées en rotation par le cadre (100) et venant coopérer avec des bras différents (210, 220 et 230) pour proposer des mises en mouvement indépendantes.

2. Ecarteur (E) selon la revendication 1, **CARACTERISE PAR LE FAIT QUE** chaque portion de roue (410, 420) est équipée d'un doigt de commande mobile (411, 421) se projetant hors du cadre (100), doigt déplaçable par l'utilisateur.

3. Ecarteur (E) selon la revendication 2, **CARACTERISE PAR LE FAIT QU'**un doigt fixe (412, 422) est fixé au cadre (100) en regard de la course de chaque doigt mobile (411, 421).

4. Ecarteur (E) selon l'une quelconque des revendications 1 à 3, **CARACTERISE PAR LE FAIT QU'**il comprend un moyen de maintien (413, 423) en position de chaque portion de roue adoptant le comportement d'un cliquet.

5. Ecarteur (E) selon l'une quelconque des revendications 1 à 3, **CARACTERISE PAR LE FAIT QUE** la première extrémité des lames (310, 320, 330) est préformée de façon à accueillir un module fonctionnel.

6. Ecarteur (E) selon la revendication 5, **CARACTERISE PAR LE FAIT QUE** la première extrémité des lames (310, 320, 330) est préformée d'au moins un orifice (311) débouchant sur la surface interne de la lame.

7. Ecarteur (E) selon l'une quelconque des revendications 1 à 6, **CARACTERISE PAR LE FAIT QUE** les portions de roues (410, 420) sont des portions de roues dentées.

8. Ecarteur (E) selon la revendication 7, **CARACTERISE PAR LE FAIT QUE** chaque bras (210, 220, 230) est monté glissant par rapport au cadre (100) et est aménagé d'une lumière longitudinale oblongue préformée sur un côté d'une crémaillère, cette crémaillère coopère avec un pignon de petit diamètre coaxial et solidaire en rotation d'un pignon de plus grand diamètre monté pivotant sur le cadre (100) et coopérant avec une portion de roue dentée.

9. Ecarteur (E) selon l'une quelconque des revendications 1 à 8, **CARACTERISE PAR LE FAIT QUE** la liaison entre la première extrémité des lames (310, 320, 330) et la deuxième extrémité des bras (210, 220, 230) autorise une inclinaison.

10. Ecarteur (E) selon l'une quelconque des revendications 1 à 9, **CARACTERISE PAR LE FAIT QU'**au moins les parties supérieures des bords longitudinaux des lames (310, 320, 330) présentent des profils complémentaires d'emboîtement optimisant la liaison entre elles une fois en contact.

11. Ecarteur (E) selon l'une quelconque des revendications 1 à 10, **CARACTERISE PAR LE FAIT QUE** les lames (310, 320, 330) sont fabriquées dans un matériau radiotransparent.

12. Ecarteur (E) selon l'une quelconque des revendications 1 à 10, **CARACTERISE PAR LE FAIT QUE** les lames sont en titane et leur épaisseur (310, 320, 330) est affinée afin de les rendre radiotransparentes.

## Patentansprüche

1. Chirurgischer Retraktor (E), umfassend
- einen Rahmen (100), der eine mittige Öffnung (110) aufweist,
- Arme (210, 220, 230), die eine Translationsbewegung ausführen können,
- je ein Blatt (310, 320, 330), das mit den Armen in Verbindung steht,
wobei die Arme (210, 220, 230) zwei Enden aufweisen:
ein erstes Ende, das zu Führungs- und Antriebszwecken mit dem Rahmen (100) zusammenwirkt, und ein zweites Ende, das sich in der mittigen Öffnung (110) bewegt,
wobei die Blätter (310, 320, 330) zwei Enden aufweisen:
ein erstes Ende, das mit dem zweiten Ende eines Arms (210, 220, 230) in Verbindung steht, und ein zweites Ende, das dazu bestimmt ist, mit den Geweben des Patienten zusammenzuwirken,
**DADURCH GEKENNZEICHNET, DASS** er zwei unabhängige nicht-koaxiale Radabschnitte (410, 420) umfasst, deren Drehbewegung von dem Rahmen (100) geführt wird und die mit verschiedenen Armen (210, 220 und 230) zusammenwirken, um diese unabhängig voneinander in Bewegung setzen zu können.

2. Retraktor (E) nach Anspruch 1, **DADURCH GEKENNZEICHNET, DASS** jeder der Radabschnitte (410, 420) mit einem beweglichen Steuerungsstift (411, 421) ausgestattet ist, der sich außenseitig des Rahmens (100) erstreckt, wobei der Stift vom Anwender bewegt werden kann.

3. Retraktor (E) nach Anspruch 2, **DADURCH GEKENNZEICHNET, DASS** ein fester Stift (412, 422) gegenüber der Laufstrecke jedes der beweglichen Stifte (411, 421) am Rahmen (100) befestigt ist.

4. Retraktor (E) nach einem beliebigen der Ansprüche 1 bis 3, **DADURCH GEKENNZEICHNET, DASS** er je ein Haltemittel (413, 423) umfasst, das bei den Radabschnitten angeordnet ist und sich wie eine Einrastvorrichtung verhält.

5. Retraktor (E) nach einem beliebigen der Ansprüche 1 bis 3, **DADURCH GEKENNZEICHNET, DASS** das erste Ende der Blätter (310, 320, 330) zur Aufnahme eines Funktionsmoduls vorgeformt ist.

6. Retraktor (E) nach Anspruch 5, **DADURCH GEKENNZEICHNET, DASS** das erste Ende der Blätter (310, 320, 330) derart vorgeformt ist, dass es mindestens eine Öffnung (311) aufweist, die in die Innenfläche des Blattes einmündet.

7. Retraktor (E) nach einem beliebigen der Ansprüche 1 bis 6, **DADURCH GEKENNZEICHNET, DASS** es sich bei den Radabschnitten (410, 420) um Zahnradabschnitte handelt.

8. Retraktor (E) nach Anspruch 7, **DADURCH GEKENNZEICHNET, DASS** jeder der Arme (210, 220, 230) derart angebracht ist, dass er gegenüber dem Rahmen (100) gleiten kann, und mit einer länglichen längsgerichteten Aussparung versehen ist, die auf einer Seite derart vorgeformt ist, dass sie eine Zahnung aufweist, wobei diese Zahnung mit einem Ritzel von geringem Durchmesser zusammenwirkt, das koaxial und drehfest mit einem Ritzel von größerem Durchmesser verbunden ist, welches drehbar am Rahmen (100) angebracht ist und mit einem Zahnradabschnitt zusammenwirkt.

9. Retraktor (E) nach einem beliebigen der Ansprüche 1 bis 8, **DADURCH GEKENNZEICHNET, DASS** die Verbindung zwischen dem ersten Ende der Blätter (310, 320, 330) und dem zweiten Ende der Arme (210, 220, 230) eine Neigung ermöglicht.

10. Retraktor (E) nach einem beliebigen der Ansprüche 1 bis 9, **DADURCH GEKENNZEICHNET, DASS** zumindest die oberen Teilbereiche der Längskanten der Blätter (310, 320, 330) komplementäre Steckverbindungsprofile aufweisen, welche ihre gegenseitige Verbindung optimieren, sobald sie einander berühren.

11. Retraktor (E) nach einem beliebigen der Ansprüche 1 bis 10, **DADURCH GEKENNZEICHNET, DASS** die Blätter (310, 320, 330) aus einem radiotransparenten Material gefertigt sind.

12. Retraktor (E) nach einem beliebigen der Ansprüche 1 bis 10, **DADURCH GEKENNZEICHNET, DASS** die Blätter aus Titan sind und ihre Dicke (310, 320, 330) verringert ist, damit sie radiotransparent sind.

## Claims

1. Surgical retractor (E) comprising:
- a frame (100) having a central opening (110),
- arms (210, 220, 230) which are movable in translation,
- a blade (310, 320, 330) associated with each arm,
the arms (210, 220, 230) having two ends:
a first end engaging with the frame (100) for guiding and driving purposes and a second end operating in the central opening (110),
the blades (310, 320, 330) having two ends:
a first end connected to the second end of an arm (210, 220, 230) and a second end intended to engage with the tissue of the patient,
**CHARACTERISED BY** THE FACT THAT it comprises two independent wheel portions (410, 420) guided in rotation by the frame (100) and engaging with different arms (210, 220 and 230) to propose independent movements.

2. Retractor (E) according to claim 1, **CHARACTERISED BY** THE FACT THAT each wheel portion (410, 420) is equipped with a movable control finger (411, 421) projecting outside of the frame (100), finger movable by the user.

3. Retractor (E) according to claim 2, **CHARACTERISED BY** THE FACT THAT a fixed finger (412, 422) is fixed to the frame (100) facing the stroke of each movable finger (411, 421).

4. Retractor (E) according to any one of claims 1 to 3, **CHARACTERISED BY** THE FACT THAT it comprises a means (413, 423) for holding each wheel portion in position, adopting the behaviour of a pawl.

5. Retractor (E) according to any one of claims 1 to 3, **CHARACTERISED BY** THE FACT THAT the first end of the blades (310, 320, 330) is preformed so as to house a functional module.

6. Retractor (E) according to claim 5, **CHARACTERISED BY** THE FACT THAT the first end of the blades (310, 320, 330) is preformed with at least one orifice (311) opening onto the internal surface of the blade.

7. Retractor (E) according to any one of claims 1 to 6, **CHARACTERISED BY** THE FACT THAT the wheel portions (410, 420) are gearwheel portions.

8. Retractor (E) according to claim 7, **CHARACTERISED BY** THE FACT THAT each arm (210, 220, 230) is slidingly mounted with respect to the frame (100) and is provided with an oblong longitudinal lumen preformed on a side of a rack, this rack engages with a pinion of small coaxial diameter and integral in rotation with a pinion of a larger diameter pivotingly mounted on the frame (100) and engaging with a gearwheel portion.

9. Retractor (E) according to any one of claims 1 to 8, **CHARACTERISED BY** THE FACT THAT the connection between the first end of the blades (310, 320, 330) and the second end of the arms (210, 220, 230) enables an inclination.

10. Retractor (E) according to any one of claims 1 to 9, **CHARACTERISED BY** THE FACT THAT at least the upper parts of the longitudinal edges of the blades (310, 320, 330) have complementary interlocking profiles optimising the connection together once in contact.

11. Retractor (E) according to any one of claims 1 to 10, **CHARACTERISED BY** THE FACT THAT the blades (310, 320, 330) are made of a radiotransparent material.

12. Retractor (E) according to any one of claims 1 to 10, **CHARACTERISED BY** THE FACT THAT the blades are made of titanium and their thickness (310, 320, 330) is refined in order to make them radiotransparent.
